# EUROPEAN PATENT SPECIFICATION

(11) **EP 4 114 329 B1**
(45) Date of publication and mention of the grant of the patent: **22.04.2026**
(21) Application number: 21764204.0
(22) Date of filing: 04.03.2021
(51) Int. Cl.: A61F 11/00, A61K 9/00, A61K 31/00, A61P 1/08, A61P 5/24, A61P 13/00, A61P 15/12, A61P 25/00, A61F 11/08, A61M 31/00

(54) **AN EARPLUG FOR ADMINISTERING A FLUID AGENT INTO AN EAR CANAL**
OHRSTÖPSEL ZUR VERABREICHUNG EINES FLÜSSIGEN MITTELS IN EINEN GEHÖRGANG
BOUCHON D'OREILLE POUR L'ADMINISTRATION D'UN AGENT FLUIDE DANS UN CANAL AUDITIF

(30) Priority: 05.03.2020 SE 2050253
(43) Date of publication of application: 11.01.2023
(73) Proprietor: ViaAuris AB, 236 34 Höllviken (SE)
(72) Inventor: ROSELL, Urban, 236 33 Höllviken (SE); SVENSSON, Nicolas, 236 34 Höllviken (SE); CAMPBELL, Erik, 236 31 Höllviken (SE)
(74) Representative: Ström & Gulliksson AB
(86) International application number: PCT/SE2021/050194
(87) International publication number: WO 2021/177889

(56) References cited:
- EP-A1- 3 639 798
- WO-A1-2017/066297
- WO-A1-93/07870
- CN-A- 102 178 997
- CN-A- 110 251 786
- CN-Y- 2 358 855
- CN-Y- 2 482 400
- US-A- 5 674 196
- US-A1- 2006 253 087
- US-A1- 2013 338 639
- US-B1- 6 401 859
- US-B2- 8 956 333

## Description

### Technical Field

The present invention generally relates to earplugs. More particularly, the present invention generally relates to an earplug for administering fluid to an ear canal of a person.

### Background

As is well known, many people have a need for treatment or relieve of different diseases, disorders or conditions by administration of different medicaments or agents. Known devices, administration methods and compositions are however in some cases not as efficient, convenient or reliable as desired.

In light of the observations above, the present inventors have realized that there is room for improvements when it comes to a device for treating or relieving medical or health conditions.

WO2017066297A1 discloses a device for the dispensing of liquids. In one design, the device comprises a first hollow bulb coupled to a second hollow bulb, wherein the first hollow bulb is compressible; and a separator element disposed between the first hollow bulb and the second hollow bulb, wherein the separator element defines a passage; wherein the second hollow bulb defines an opening.

### Summary

It is accordingly an object of the invention to eliminate or alleviate at least some of the problems or drawbacks referred to above.

A first aspect of the present invention therefore is an earplug for administering a fluid agent into an ear canal. The earplug comprises a first part and a second part. The first part is adapted for insertion in the ear canal and comprises an elongated member which has a chamber for containing the fluid agent. The chamber has at least one outlet at a proximal end of the elongated member. The malleable member is disposed laterally of the elongated member.

The second part comprises a piston having a first end, which is insertable in the elongated member, and a second end which has a push member. The piston is displaceable from a first position distally of the chamber to a second position within the chamber, such that the fluid agent is injected from the chamber through the outlet into the ear canal.

Accordingly, when the earplug is positioned in the ear canal of a person prior to injection of said fluid agent into the ear canal of said person, the chamber and the fluid agent contained therein are positioned in the ear canal of said person.

The provision of an earplug as disclosed herein will solve or at least mitigate one or more of the problems or drawbacks identified in the Background section of this document. These and other aspects, objectives, features and advantages of the invention and its disclosed embodiments will appear from the following detailed disclosure, from the attached dependent claims as well as from the drawings. A key feature of the earplug is the user convenience it offers. The user may simply insert the earplug into the ear canal, actuate the push member to inject the fluid agent, and then leave the earplug in place for the duration of the treatment. This will keep the injected fluid agent in place in the ear canal (as opposed to leaking out) and thus increase the efficiency of the treatment.

A related example, not encompassed by the claimed invention but useful for the understanding thereof, is a method of administering a liquid medicament to a person, the method comprising:
providing an earplug, the earplug having an internal container for the liquid medicament, and an injector mechanism;
inserting the earplug in an ear canal of the person;
actuating the injector mechanism to cause injection of the liquid medicament from the internal container of the earplug to the ear canal of the person;
leaving the earplug in place in the ear canal for a duration of treatment; and
removing the earplug from the ear canal when the duration of treatment has lapsed.

The earplug used in the method according to the related example may advantageously be the earplug as referred to above for the first aspect of the present invention.

Further related examples, not encompassed by the claimed invention but useful for the understanding thereof, are compositions for treatment of various diseases, disorders and conditions.

Generally, all terms used in the claims are to be interpreted according to their ordinary meaning in the technical field, unless explicitly defined otherwise herein.

All references to "a/an/the [element, device, component, means, step, etc.]" are to be interpreted openly as referring to at least one instance of the element, device, component, means, step, etc., unless explicitly stated otherwise. The steps of any method disclosed herein do not have to be performed in the exact order disclosed, unless explicitly stated.

A reference to an entity being "designed for" doing something, or "capable of" doing something in this document is intended to mean the same as the entity being "arranged for", "configured for" or "adapted for" doing this very something, and vice versa.

### Brief Description of the Drawings

Fig 1 illustrates an embodiment of an earplug for administering a fluid agent into an ear canal.
Fig 2 is a sectional isometric view of the earplug in Fig 1, a twistable endcap having been removed and showing an internal piston in a first position.
Fig 3 is a sectional isometric view of the earplug in Fig 1, showing the piston in a second position.
Fig 4 is a sectional side view of the earplug, showing the internal piston in the first position.
Fig 5 is a sectional side view of the earplug, showing the internal piston in the second position.
Fig 6 is an exploded isometric view of a flexible cover and a malleable member being parts of the earplug.
Fig 7 is an exploded isometric view of the piston and an elongated member being other parts of the earplug.
Fig 8 is a detailed sectional view of an arrangement for retaining the piston in the first position.
Fig 9 is a diagram showing results from a test performed with the earplug.

### Detailed Description

Embodiments of the invention will now be described with reference to the accompanying drawings. The invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the particular embodiments illustrated in the accompanying drawings is not intended to be limiting of the invention. In the drawings, like numbers refer to like elements. In this detailed description the terms proximal and distal are used in relation to the person on which an earplug is applied. This means that proximal is used to indicate towards the person, while distal is used to indicate away from the person. The terms lateral and central are used in relation to the central axis of an earplug - positioned in the ear canal of a person or to be positioned in an ear canal of a person.

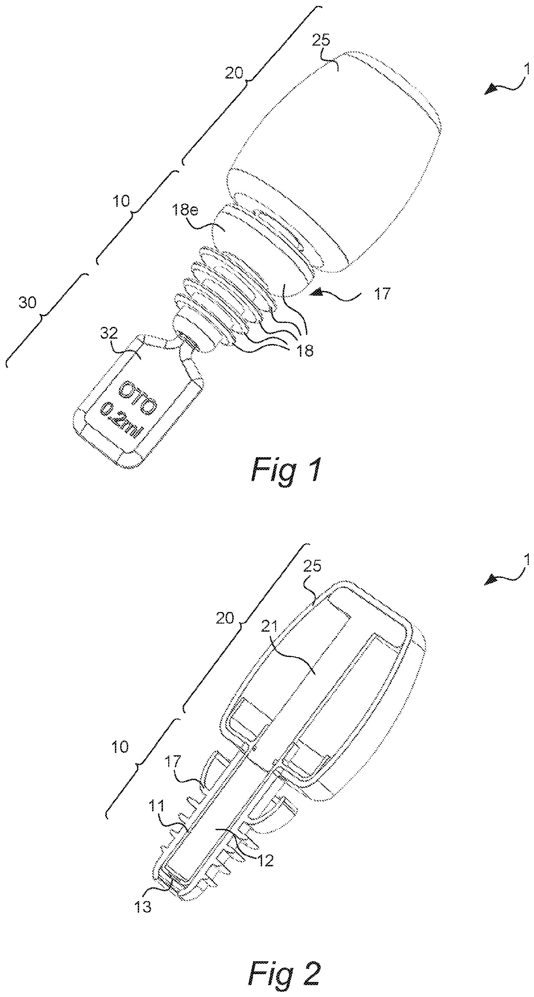

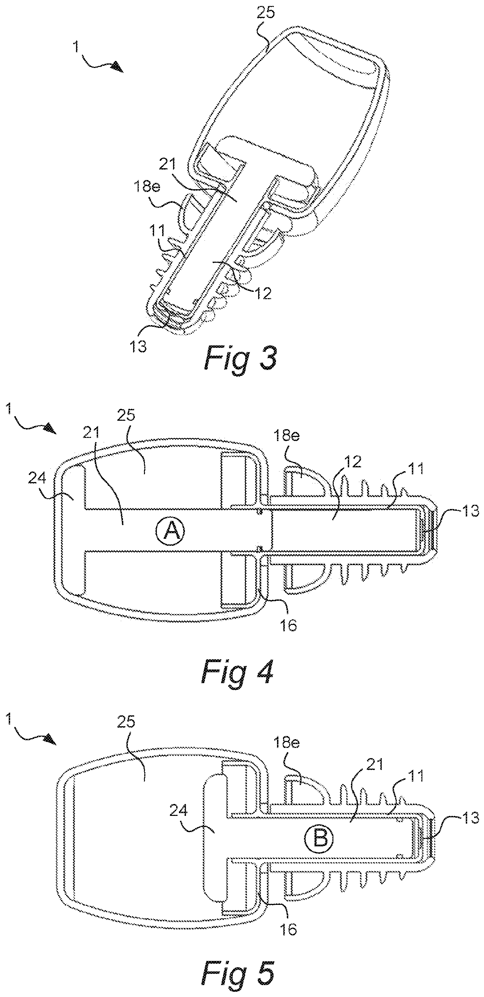

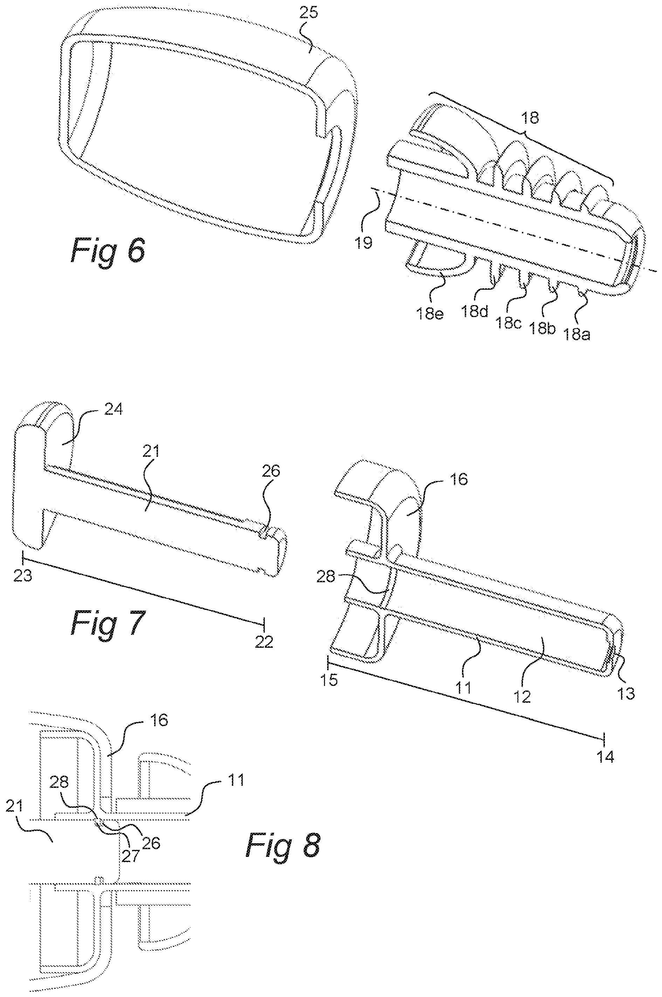

Figs. 1 to 8 disclose an earplug 1 according to an embodiment of the present invention. The earplug 1 comprises a first part 10 adapted for insertion in the ear canal of a person. The first part 10 comprises an elongated member 11. The elongated member 11 is provided with a chamber 12. The chamber 12 is configured to contain a fluid agent, to be administered, such as injected into an ear canal of a person. The chamber 12 is a central chamber positioned centrally of an outer circumferential wall of the elongated member 11. When the earplug 1 is positioned in the ear canal of a person, the chamber 12, comprising the fluid agent, is positioned in the ear canal of the person. This makes the earplug 1 compact, and decreases spill of the fluid agent. The chamber 12 has an outlet 13. The outlet 13 is arranged at the proximal end of the elongated member 11. The outlet 13 may be formed by a circumferential flange, extending centrally from the outer wall of the elongated member 11. The flange creates a constriction, which creates a pressure drop at the outlet 13 upon administration, increasing the velocity of the fluid during administration. The elongated member 11 is provided laterally with a malleable member 17. The malleable member 17 conforms to the inner surface of the ear canal, to seal off the ear canal from the ambiance and prevents administered fluid to leak out of the ear canal. The malleable member may comprise a plurality of resilient flanges 18. The resilient flanges 18 extend laterally. The resilient flanges 18 may be distributed longitudinally in a longitudinal main direction, i.e. proximodistal direction, of the earplug 1. At least the most distal resilient flange 18d may be curve shaped, convex from proximal perspective and concave from a distal perspective, which improves adaptation to different persons and the sealing effect.

The radial/lateral extension of the resilient flanges 18 may decrease along the elongated member in the proximal direction. Hence, the most proximal resilient flange 18a may have the smallest radial/lateral extension, and the most distal resilient flange 18e may have the greatest radial/lateral extension. This configuration facilitates insertion of the earplug 1, and adapts to dimensional variations of ear canals in different persons.

The malleable member 17 may be of a suitable rubber material, such as a silicone rubber, neoprene, ethylene propylene diene monomer rubber, and the like. These materials conform well with the ear canal of a person and provides good sealing effect towards the ear canal, such that the fluid does not leak out from the ear canal into the surroundings. In this way the earplug 1 may be used to administer ear wax dissolving agents, such as Debrox, Kyrosol, Revaxör, Waxsol, Earclear, comprising active agents, such as carbamide peroxide, docusate sodium,

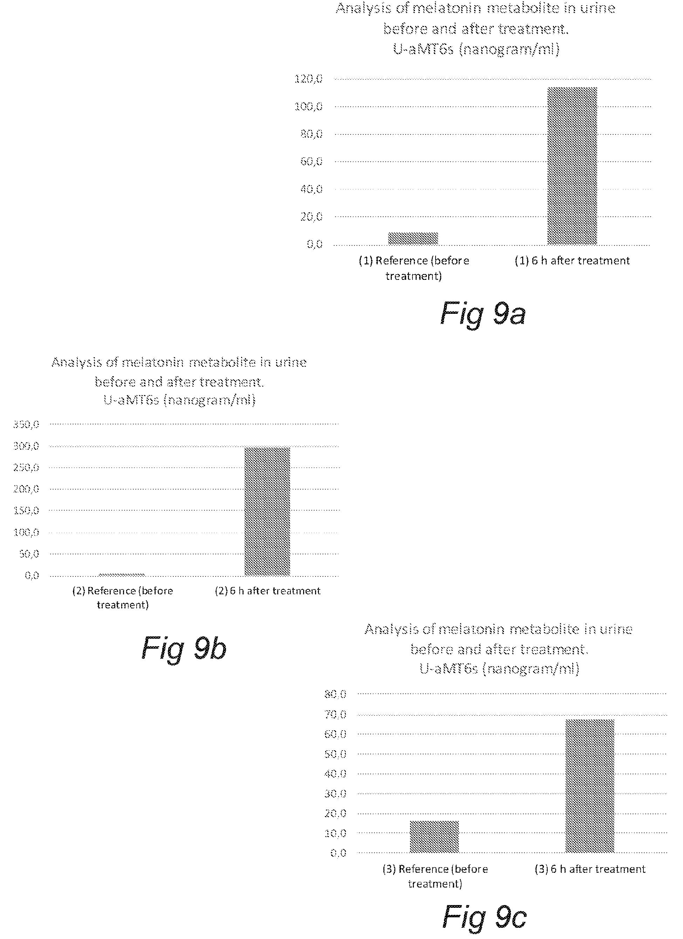

An additional benefit of the malleable member 17 in embodiments of the invention is that it may also serve to acoustically seal off the ear canal and thus the inner ear from the surroundings. In other words, the earplug may have an additional function to reduce ambient noise, which may be particularly beneficial in embodiments where the fluid agent is an agent, such as melatonin, that seeks to relieve the user from insomnia or stress disorders. The user may then enjoy two relieving effects from using the earplug: a first effect caused by ingestion of the fluid agent into the user's bloodstream through the thin skin in the ear canal, and a second effect obtained by reduction of ambient noise. As can be seen in Fig. 9, the inventors have surprisingly found that medical agents can be administered through the ear canal, entering the blood stream through the skin in an effective way. In the examples disclosed in Fig. 9, a 56 year old male, another 56 year old male, as well as a 66 year old male were administered 2 mg melatonin in the ear canal. Prior to the administration a urine sample was taken to establish a reference value of the melatonin metabolite 6-Sulfatoxymelatonin (aMT6s) at a certain time. The reference values were measured to 8.3 ng/ml, 3.8 ng/ml, and 16.3 ng/ml of aMT6s, as disclosed in the left bars in Figs. 9a, 9b, and 9c. Six hours after administration of the melatonin, new urine samples were taken. The amounts of aMT6s were again measured, and the amounts were set to 114.0 ng/ml, 297.0 ng/ml, and 67.7 ng/ml of aMT6s, as disclosed in the right bars in Figs. 9a, 9b, and 9c. Even when taking natural variability during a day, this increase shows a statistically significant increase, that can only be the result of melatonin uptake through the ear canal. Since melatonin is entering the bloodstream through the ear canal, there is no reason to believe that other active agents, capable of penetrating skin, i.e. transdermal drug delivery (TDD), should not be able to be administered through the ear canal. TDD is a painless method of delivering drugs systemically by applying a drug formulation onto intact and healthy skin. The drug initially penetrates through the stratum corneum and then passes through the deeper epidermis and dermis without drug accumulation in the dermal layer. When drug reaches the dermal layer, it becomes available for systemic absorption via the dermal microcirculation. TDD has many advantages over other conventional routes of drug delivery. It can provide a non-invasive alternative to parenteral routes, thus circumventing issues such as needle phobia. A large surface area of skin and ease of access allows many placement options on the skin for transdermal absorption. Furthermore, the pharmacokinetic profiles of drugs are more uniform with fewer peaks, thus minimizing the risk of toxic side effects. It can improve patient compliance due to the reduction of dosing frequencies and is also suitable for patients who are unconscious or vomiting, or those who rely on self-administration. TDD avoids pre-systemic metabolism, thus improving bioavailability. With reference to the use of the skin as a novel site for vaccination strategies, this organ is known to be replete with dendritic cells in both the epidermal and dermal layers, which play a central role in immune responses making TDD an attractive vaccination route for therapeutic proteins and peptides. The requirement for an inexpensive and non-invasive means of vaccination, especially in the developing world, has given rise to substantial research focused on the development of simple, needle-free systems, such as TDD, for vaccination purposes. There are two possible routes of drug penetration across the intact skin, namely the transepidermal and transappendegeal pathways. The transepidermal pathway involves the passage of molecules through the stratum corneum, an architecturally diverse, multi-layered and multi-cellular barrier. Transepidermal penetration can be termed intra- or inter-cellular. The intra-cellular route through corneocytes, terminally differentiated keratinocytes, allows the transport of hydrophilic or polar solutes. Transport via inter-cellular spaces allows diffusion of lipophilic or non-polar solutes through the continuous lipid matrix. The transappendegeal route involves the passage of molecules through sweat glands and across the hair follicles.

As a consequence, ear canal TDD should be applicable and made evident by the example disclosed in Fig. 9 for numerous active agents, such as nicotine; opioids, such as fentanyl and buprenorphine; nitroglycerin; scopolamine; clonidine; vitamins, such as vitamin B12; selegiline; 5-Hydroxytryptophan (5-HTP); hormones, such as melatonin, estradiol, ethinylestradiol, testosterone, progesterone, norethisterone acetate, norelgestromin, levonorgestrel, etc; agents acting on muscarinic receptors, such as tolterodine and oxybutynin; norepinephine-dopamine reuptake inhibitors, such as methylphenidate; dopamine agonists, such as rotigotine; cholinesterase inhibitors, such as rivastigmine; serotonin receptor antagonists, such as granisetron, etc. As such ear canal TDD with the ear plug 1 according to the present invention can be used to treat an indication selected from the group comprising sleep disorders, such as insomnia; stress disorders; nicotine dependency; pain; heart disorders; vitamin insufficiency; motion sickness; blood pressure deviations; mood disorders, such as depression, anxiety, and attention deficit hyperactivity disorder (ADHD); hormone deviations, such as menopausal disorders and birth control; weight gain; urination disorders, such as frequent urination, urinary incontinence, or urinary urgency; Parkinson's disease (PD); Alzheimer's disease; and nausea and vomiting.

The earplug 1 further comprises a second part 20. The second part 20 is arranged distally of the first part 10. The second part 20 comprises a piston 21. The piston 21 has a proximal first end 22, which is configured to be inserted into the elongated member 11. The piston 21 is configured to be inserted into the distal end of the elongated member 11, and into the chamber 12. The outer/lateral end surface of the proximal end of the piston 21 conforms to the shape and dimensions of the inner surface of elongated member 11, defining the chamber 12. In this way proximal movement of the piston 21 will effectively be able to expel fluid from the earplug 1 via proximal movement within the chamber 12, such that fluid exits the outlet 13 of the earplug 1. At the distal end of the second member 20, the second member 20 is provided with a push member 24. The push member 24 is disclosed in the present embodiments as a push plate 24. The piston 21 is, in accordance with above, displaceable from a first position A distally of the chamber 12 to a second position B within the chamber 12, such that the fluid agent is injected from the chamber 12 through the outlet 13 into the ear canal.

The second part 20 comprises a flexible cover 25. The flexible cover 25 is attached at its proximal end to the first part 10. The flexible cover 25 encapsulates the piston 21 and the push member 24 in the first position A. In this manner, the flexible cover 25 protects the piston 21 and the push member 24, and encapsulates the distal end of the first member 10, such that the risk of tampering with fluid in the chamber 13 is decreased. The flexible cover 25 has a flexibility allowing for proximal movement of the piston 21 upon an applied proximal push force at on the piston 21 via the flexible cover 25. Pushing onto the flexible cover 25 and the piston 21 displaces the piston 21 proximally from the first position A to the second position B, to inject the fluid into the ear canal of a person. After displacement of the piston 21 in the proximal direction and injecting fluid into the ear canal of a person, the flexible cover 25 remains attached to the first part 10 and continues to extend distally from the first part 10. In this position the flexible cover may serve as a handle for subsequent removal of the earplug 1 from the ear canal.

At the distal end of the elongated member 11 of the first part 10 an end cup 16 may be arranged. The end cup 16 allows for proximal back-side surface for the flexible cover 25 to attach to, such that the flexible cover 25 may encapsulate the piston 21 and the distal opening of the chamber 13 in the above described manner. Further, the attachment of the flexible cover 25 on the proximal side of the end cup 16 decreases the risk of the flexible cover 25 to detach upon the removal force in the distal direction. Also, the end cup 16 is configured to receive the push member 24, such as a push plate, when the piston 21 has been displaced from position A to position B. When the end cup 16 receives the push member 24, the distal end surface of the push member 24 may be aligned with or positioned proximally of the distal end of the circumferential socket of the end cup 16. In this way, the end cup 16 may facilitate maintenance of the piston 21 in position B.

The piston 21 may be provided with a sealing member 27 at its proximal end zone. The sealing member 27 may comprise an o-ring gasket that sits in a circumferential groove 26. In this way displacement of the sealing member 27 in the proximodistal direction upon displacement of the piston 21 from position A to B may be avoided. Correspondingly, the elongated member 11 may have a circumferential groove 28 in the chamber 13, at an distal end zone 15 of the chamber 13. The circumferential groove 28 is configured to receive the lateral part of the sealing member 27 in the first position A. This means that there is a need of an initiation force to displace the piston 21 in the proximal direction, which decreases the risk of unintentional ejection of fluid from the chamber 13.

At the proximal end of the earplug 1 a third part 30 may be arranged. Then, the first part 10 is disposed between the second part 20 and the third part 30. The third part 30 is then arranged proximally of the first part 10 and the second part 20 is arranged distally of the first part 10. The third part 30 is then a removable obstructor 30 of the outlet 13 of the elongated member 11. The removable obstructor 30 prevents fluid from being ejected from the chamber 13 and it prevents contamination of the fluid. The removable obstructor 30 may be a twistable cap. Then, there may be a weakening between the obstructor 30 and the first part 10, and the obstructor extending proximally in for of a plate with side surfaces parallel to the central axis of the earplug 1, for easy manual twisting action to break the weakening and revealing the outlet 13.

A method of administering a liquid medicament to a person will now be described. The method, not encompassed by the claimed invention but useful for the understanding thereof, involves the following steps:
i) providing an earplug, the earplug having an internal container for the liquid medicament, and an injector mechanism;
ii) inserting the earplug in an ear canal of the person;
iii) actuating the injector mechanism to cause injection of the liquid medicament from the internal container of the earplug to the ear canal of the person;
iv) leaving the earplug in place in the ear canal for a duration of treatment; and
v) removing the earplug from the ear canal when the duration of treatment has lapsed.

The earplug (1) as described with reference to Fig 1 to Fig 8 may advantageously be used in this method.

The method is advantageously a method for treating an indication selected from the group consisting of sleep disorders, such as insomnia; stress disorders; nicotine dependency; pain; heart disorders; vitamin insufficiency; motion sickness; blood pressure deviations; mood disorders, such as depression, anxiety, and attention deficit hyperactivity disorder (ADHD); hormone deviations; weight gain; urination disorders, such as frequent urination, urinary incontinence, or urinary urgency; Parkinson's disease (PD); Alzheimer's disease; and nausea and vomiting

The invention has been described above in detail with reference to embodiments thereof. However, as is readily understood by those skilled in the art, other embodiments are equally possible within the scope of the present invention, as defined by the appended claims.

## Claims

1. An earplug (1) for administering a fluid agent into an ear canal, the earplug comprising:
a first part (10) adapted for insertion in the ear canal, said first part comprising:
an elongated member (11) having a chamber (12) for containing the fluid agent, the chamber (12) having at least one outlet (13) at a proximal end (14) of the elongated member (11); and
a malleable member (17) disposed laterally of the elongated member (11);
a second part (20) comprising a piston (21), the piston having:
a first end (22) which is insertable in the elongated member (11); and
a second end (23) which has a push member (24),
wherein the piston (21) is displaceable from a first position (A) distally of the chamber (12) to a second position (B) within the chamber (12), such that the fluid agent is injected from the chamber (12) through the outlet (13) into the ear canal.

2. The earplug (1) as defined in claim 1, wherein, when the earplug (1) is adapted to be positioned in the ear canal of a person prior to injection of said fluid agent into the ear canal of said person, so that the chamber (12) and the fluid agent contained therein are positioned in the ear canal of said person.

3. The earplug (1) as defined in claim 1 or 2, wherein the second part (20) further comprises a flexible cover (25) attached to the first part (10) and being configured for encapsulating the piston (21) and the push member (24) in the first position (A).

4. The earplug (1) as defined in claim 3, wherein the flexible cover (25) is further configured for allowing the piston (21) to be displaced to the second position (B) by actuation of the push member (24) and for remaining attached to the first part (10) also in the second position (B), the flexible cover (25) thereby serving as a handle for subsequent removal of the earplug (1) from the ear canal.

5. The earplug (1) as defined in any preceding claim, wherein the elongated member (11) of the first part (10) further comprises an end cup (16) at a distal end (14) thereof, wherein the flexible cover (25) encapsulates the end cup (16) and wherein the end cup (16) is shaped and configured for retaining the flexible cover (25) attached to the first part (10).

6. The earplug (1) as defined in any preceding claim, wherein the malleable member (17) is made of a material selected from the group consisting of silicone rubber, neoprene, and ethylene propylene diene monomer rubber.

7. The earplug (1) as defined in any preceding claim, wherein the malleable member (17) has a plurality of resilient flanges (18) distributed in a longitudinal main direction (19) of the malleable member (17).

8. The earplug (1) as defined in claim 7, wherein the plurality of resilient flanges (18; 18a-18e) of the malleable member 17) have decreasing radial sizes, a first flange (18a) at or near the proximal end (14) of the elongated member (11) having a smallest radial size and a last flange (18e) at or near the distal end (15) of the elongated member (11) having a largest radial size.

9. The earplug (1) as defined in any preceding claim, wherein the piston (21) has a sealing member (27) near or at the first end (22).

10. The earplug (1) as defined in claim 9, wherein the sealing member (27) comprises an o-ring gasket and wherein the piston (21) has a circumferential groove (26) near or at the first end (22) for receiving the o-ring gasket.

11. The earplug (1) as defined in claim 9 or 10, wherein the elongated member (11) has a circumferential groove (28) near or at the distal end (15), the circumferential groove (28) being configured for receiving at least a part of the sealing member (27) of the piston (21) in the first position (A) of the piston (21).

12. The earplug (1) as defined in any preceding claim, the earplug (1) further comprising a third part (30), the first part (10) being disposed between the second and third parts (20, 30) of the earplug (1), the third part (30) being a removable obstructor of the outlet (13) of the elongated member (11).

13. The earplug (1) as defined in claim 12, wherein the third part (30) comprises a twistable cap.

## Patentansprüche

1. Ohrstöpsel (1) zur Verabreichung eines flüssigen Mittels in einen Gehörgang, wobei der Ohrenstöpsel folgendes aufweist:
ein erstes Teil (10), das zur Einführung in den Gehörgang angepasst ist und folgendes aufweist:
ein längliches Element (11), das eine Kammer (12) hat, die dazu ausgebildet ist, das flüssige Mittel zu enthalten, wobei die Kammer (12) mindestens einen Auslass (13) an einem proximalen Ende (14) des länglichen Elements (11) hat; und
ein verformbares Element (17), das seitlich des länglichen Elements (11) angeordnet ist,
ein zweites Teil (20), das einen Kolben (21) aufweist, wobei der Kolben (21) folgendes aufweist:
ein erstes Ende (22), das einführbar in das längliche Element (11) ist, und
ein zweites Ende (23), das ein Schubelement (24) hat,
wodurch der Kolben (21) aus einer ersten Position (A) distal der Kammer (12) in eine zweite Position (B) innerhalb der Kammer (12) bewegbar ist, sodass das flüssige Mittel aus der Kammer (12) durch den Auslass (13) in den Gehörgang injiziert wird.

2. Ohrstöpsel (1) gemäß Anspruch 1, wobei der Ohrstöpsel (1) angepasst ist, um vor der Injektion des flüssigen Mittels im Gehörgang einer Person so positioniert zu werden, dass die Kammer (12) und das sich darin befindende flüssige Mittel sich im Gehörgang der Person befinden.

3. Ohrstöpsel (1) gemäß Anspruch 1 oder 2, wobei das zweite Teil (20) zusätzlich eine flexible Abdeckung (25) aufweist, die an dem ersten Teil (10) befestigt ist und dazu ausgebildet ist, den Kolben (21) und das Schubelement (24) in der ersten Position (A) zu umhüllen.

4. Ohrstöpsel (1) gemäß Anspruch 3, wobei die flexible Abdeckung (25) ferner so ausgebildet ist, dass der Kolben (21) durch Betätigen des Schubelements (24) in die zweite Position (B) bewegt werden kann, und auch in Position (B) an dem ersten Teil (10) befestigt bleibt, sodass die flexible Abdeckung (25) dabei als Handgriff für die anschließende Entfernung des Ohrstöpsels (1) aus dem Gehörgang dient.

5. Ohrstöpsel (1) gemäß einem der vorhergehenden Ansprüche, wobei das längliche Element (11) des ersten Teils (10) zusätzlich ein Aufnahmeendstück (16) an seinem distalen Ende (14) aufweist, wobei die flexible Abdeckung (25) das Aufnahmeendstück (16) umhüllt, und wobei das Aufnahmeendstück (16) geformt und ausgebildet ist, um die an dem ersten Teil (10) befestigte flexible Abdeckung (25) zurückzuhalten.

6. Ohrstöpsel (1) gemäß einem der vorhergehenden Ansprüche, wobei das verformbare Element (17) aus einem Material besteht, das ausgewählt ist aus der Gruppe bestehend aus Silikonkautschuk, Neopren und Ethylen-Propylen-Dien-Monomer-Kautschuk.

7. Ohrstöpsel (1) gemäß einem der vorhergehenden Ansprüche, wobei das verformbare Element (17) eine Mehrzahl an elastischen Lippen (18) aufweist, die in Längsrichtung des verformbaren Elements (17) verteilt sind.

8. Ohrstöpsel (1) gemäß Anspruch 7, wobei die Mehrzahl an elastischen Lippen (18; 18a-e) des verformbaren Elements (17) abnehmende radiale Abmessungen aufweisen, wobei eine erste Lippe (18a) am oder in der Nähe des proximalen Endes (14) des länglichen Elements (11) die kleinste radiale Abmessung und eine letzte Lippe (18e) am oder in der Nähe des distalen Endes (15) des länglichen Elements (11) die größte radiale Abmessung aufweisen.

9. Ohrstöpsel (1) gemäß einem der vorhergehenden Ansprüche, wobei der Kolben (21) ein Dichtungselement (27) am oder in der Nähe des ersten Endes (22) aufweist.

10. Ohrstöpsel (1) gemäß Anspruch 9, wobei das Dichtungselement (27) ein O-Ring-Dichtungselement aufweist und wobei der Kolben (21) eine umlaufende Nut (26) am oder in der Nähe des ersten Endes (22) aufweist, um das O-Ring-Dichtungselement aufzunehmen.

11. Ohrstöpsel (1) gemäß Anspruch 9 oder 10, wobei das längliche Element (11) eine umlaufende Nut (28) am oder in der Nähe des distalen Endes (15) aufweist, wobei die umlaufende Nut (28) ausgebildet ist, um zumindest einen Teil des Dichtungselements (27) des Kolbens (21) in der ersten Position (A) des Kolbens (21) aufzunehmen.

12. Ohrstöpsel (1) gemäß einem der vorergehenden Ansprüche, wobei der Ohrstöpsel (1) ferner ein drittes Teil aufweist (30), wobei das erste Teil (10) zwischen dem zweiten (20) und dritten Teil (30) des Ohrstöpsels (1) angeordnet ist (10), wobei das dritte Teil (30) ein entfernbares Verschlusselement des Auslasses (13) des länglichen Elements (11) ist.

13. Ohrstöpsel (1) gemäß Anspruch 12, wobei das dritte Teil (30) eine drehbare Kappe aufweist.

## Revendications

1. Bouchon d'oreille (1) pour administrer un agent fluide dans un conduit auditif, le bouchon d'oreille comprenant :
une première partie (10) adaptée à l'insertion dans le conduit auditif, ladite première partie comprenant :
un organe allongé (11) présentant une chambre (12) destinée à contenir l'agent fluide, la chambre (12) présentant au moins une sortie (13) à une extrémité proximale (14) de l'organe allongé (11) ; et
un organe malléable (17) disposé latéralement par rapport à l'organe allongé (11) ;
une deuxième partie (20) comprenant un piston (21), le piston présentant :
une première extrémité (22) qui peut être insérée dans l'organe allongé (11) ;
et
une seconde extrémité (23) qui présente un organe de poussée (24),
dans lequel le piston (21) est déplaçable d'une première position (A) de manière distale par rapport à la chambre (12) à une seconde position (B) à l'intérieur de la chambre (12), de sorte que l'agent fluide soit injecté depuis la chambre (12) dans le conduit auditif, en passant par la sortie (13).

2. Bouchon d'oreille (1) selon la revendication 1, dans lequel, lorsque le bouchon d'oreille (1) est conçu pour être positionné dans le conduit auditif d'une personne avant l'injection dudit agent fluide dans le conduit auditif de ladite personne, de sorte que la chambre (12) et l'agent fluide contenu dans celle-ci soient positionnés dans le conduit auditif de ladite personne.

3. Bouchon d'oreille (1) selon la revendication 1 ou 2, dans lequel la deuxième partie (20) comprend en outre un couvercle flexible (25) fixé à la première partie (10) et étant configuré pour encapsuler le piston (21) et l'organe de poussée (24) dans la première position (A).

4. Bouchon d'oreille (1) selon la revendication 3, dans lequel le couvercle flexible (25) est en outre configuré pour permettre le déplacement du piston (21) vers la seconde position (B) par actionnement de l'organe de poussée (24) et pour rester fixé à la première partie (10) également dans la seconde position (B), le couvercle flexible (25) servant ainsi de poignée pour le retrait ultérieur du bouchon d'oreille (1) du conduit auditif.

5. Bouchon d'oreille (1) selon une quelconque revendication précédente, dans lequel l'organe allongé (11) de la première partie (10) comprend en outre une coupelle d'extrémité (16) à une extrémité distale (14) de celui-ci, dans lequel le couvercle flexible (25) encapsule la coupelle d'extrémité (16) et dans lequel la coupelle d'extrémité (16) est formée et configurée pour retenir le couvercle flexible (25) fixé à la première partie (10).

6. Bouchon d'oreille (1) selon une quelconque revendication précédente, dans lequel l'organe malléable (17) est en un matériau sélectionné dans le groupe consistant en le caoutchouc silicone, le néoprène et le caoutchouc monomère d'éthylène-propylène-diène.

7. Bouchon d'oreille (1) selon une quelconque revendication précédente, dans lequel l'organe malléable (17) présente une pluralité de brides élastiques (18) réparties dans une direction principale longitudinale (19) de l'organe malléable (17).

8. Bouchon d'oreille (1) selon la revendication 7, dans lequel la pluralité de brides élastiques (18 ; 18a-18e) de l'organe malléable (17) présentent des dimensions radiales décroissantes, une première bride (18a) au niveau ou près de l'extrémité proximale (14) de l'organe allongé (11) présentant la petite dimension radiale et une dernière bride (18e) au niveau ou près de l'extrémité distale (15) de l'organe allongé (11) présentant la plus grande dimension radiale.

9. Bouchon d'oreille (1) selon une quelconque revendication précédente, dans lequel le piston (21) présente un organe d'étanchéité (27) proche ou au niveau de la première extrémité (22).

10. Bouchon d'oreille (1) selon la revendication 9, dans lequel l'organe d'étanchéité (27) comprend un joint torique et dans lequel le piston (21) présente une rainure circonférentielle (26) proche ou au niveau de la première extrémité (22) pour recevoir le joint torique.

11. Bouchon d'oreille (1) selon la revendication 9 ou 10, dans lequel l'organe allongé (11) présente une rainure circonférentielle (28) proche ou au niveau de l'extrémité distale (15), la rainure circonférentielle (28) étant configurée pour recevoir au moins une partie de l'organe d'étanchéité (27) du piston (21) dans la première position (A) du piston (21).

12. Bouchon d'oreille (1) selon une quelconque revendication précédente, le bouchon d'oreille (1) comprenant en outre une troisième partie (30), la première partie (10) étant disposée entre les deuxième et troisième parties (20, 30) du bouchon d'oreille (1), la troisième partie (30) étant un élément d'obstruction amovible de la sortie (13) de l'organe allongé (11).

13. Bouchon d'oreille (1) selon la revendication 12, dans lequel la troisième partie (30) comprend un capuchon pivotant.
